# EUROPÄISCHE PATENTSCHRIFT

(11) **EP 2 403 360 B1**
(45) Veröffentlichungstag und Bekanntmachung des Hinweises auf die Patenterteilung: **10.08.2016**
(21) Anmeldenummer: 10706652.4
(22) Anmeldetag: 04.03.2010
(51) Int. Cl.: A23K 20/179, A23L 5/44, C07C 403/24

(54) **FORMULIERUNG VON ASTAXANTHIN-DERIVATEN UND DEREN VERWENDUNG III**
FORMULATION OF ASTAXANTHIN DERIVATIVES AND USE THEREOF III
FORMULATION DE DÉRIVÉS D'ASTAXANTHINE ET LEUR UTILISATION III

(30) Priorität: 05.03.2009 EP 09154444
(43) Veröffentlichungstag der Anmeldung: 11.01.2012
(73) Patentinhaber: BASF SE, 67056 Ludwigshafen am Rhein (DE)
(72) Erfinder: FELDTHUSEN JENSEN, Jesper, 55268 Nieder-Olm (DE); KÖPSEL, Christian, 69469 Weinheim (DE); END, Lutz, 68526 Ladenburg (DE); BRANDS, Mario, 67063 Ludwigshafen (DE); ENGEL, Robert, 67346 Speyer (DE); GOTTSCHALK, Thomas, 68165 Mannheim (DE); PELLETIER, Wolf, 76879 Ottersheim (DE)
(74) Vertreter: BASF IP Association
(86) Internationale Anmeldenummer: PCT/EP2010/052762
(87) Internationale Veröffentlichungsnummer: WO 2010/100232

(56) Entgegenhaltungen:
- EP-A2- 0 845 503
- EP-A2- 1 213 013
- EP-A2- 1 228 705
- WO-A1-03/066583
- WO-A2-2005/075385

## Beschreibung

Die vorliegende Erfindung betrifft Formulierungen von Astaxanthin-Derivaten sowie die Verwendung der Formulierungen zur Herstellung von Lösungen des Astaxanthin-Derivats in für Nahrungsmittel geeigneten Ölen sowie zur Herstellung von Futtermitteln. Die Erfindung betrifft auch ein Verfahren zur Herstellung derartiger Formulierungen und ein Verfahren zur Herstellung von Futtermitteln unter Verwendung der Formulierungen.

Astaxanthin (3,3'-Dihydroxy-ß,ß-carotin-4,4'-dion) und seine Derivate, insbesondere seine Ester, sind als Nahrungs- und Futtermittelzusatzstoffe von Interesse. So wird Astaxanthin in großem Umfang bei der Aufzucht von Speisefischen in Aquakulturen (Fischfarmen) als Futtermittelzusatz eingesetzt. Aufgrund der vitaminartigen Eigenschaft von Astaxanthin und seinen Derivaten wirken sie sich vorteilhaft auf die Gesundheit der Fische in den Aquakulturen aus und fördern deren Fruchtbarkeit. Zudem bewirken Astaxanthin und seine Derivate eine Intensivierung der Färbung von Fleisch und Haut der Fische, was nicht zuletzt aus ästhetischen und kulinarischen Gründen wünschenswert ist.

Problematisch sind die geringe Wasserlöslichkeit von Astaxanthin und seinen Derivaten und ihre damit einhergehende schlechte Bioverfügbarkeit. Aus diesem Grund können Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität vom Astaxanthin und seinen Derivaten stellt jedoch die Formulierung dieser Verbindungen eine besondere Herausforderung dar.

Für verschiedene Anwendungszwecke, insbesondere zur Futtermittelherstellung, hat es sich grundsätzlich als vorteilhaft erwiesen, Astaxanthin oder seine Derivate in Form von verdünnten Lösungen in für Nahrungsmittel geeigneten, flüssigen Ölen bereitzustellen. Diese verdünnten Lösungen können dann bei der Futtermittelherstellung verwendet werden und gewährleisten eine hinreichende Bioverfügbarkeit in dem Futtermittel. Bei der Herstellung derartiger Lösungen erweist sich die schlechte Lösungskinetik des Astaxanthins als problematisch.

Die EP 65193 beschreibt ein Verfahren zur Herstellung pulverförmiger Carotinoid-Zusammensetzungen mit Partikelgrößen < 0,5 µm, bei dem man das Carotinoid zunächst in einem flüchtigen, mit Wasser mischbaren Lösungsmittel bei erhöhter Temperatur löst, das Carotinoid aus der so erhaltenen Lösung durch rasches Eintragen in eine wässrige Lösung eines quellbaren Schutzkolloids fällt und die so erhaltene Suspension sprühtrocknet. Das pulverförmige Carotinoid ist in Wasser dispergierbar. Ölige Lösungen werden nicht beschrieben.

Die WO 00/66665 beschreibt ein ähnliches Verfahren zur Herstellung von in Wasser dispergierbaren Trockenpulvern des Astaxanthins. Als Schutzkolloid enthalten die Trockenpulver Casein. Die EP 1228705 und EP 1219292 beschreiben in Wasser dispergierbare Trockenpulver des Astaxanthins ähnlich denen der WO 00/66665, die als Schutzkolloid ein Soja-Protein enthalten. Eigene Untersuchungen der Erfinder der vorliegenden Anmeldung haben gezeigt, dass bei direkter Einarbeitung der dort beschriebenen Trockenpulver in für Nahrungsmittel geeignete Öle das Astaxanthin nicht oder nur zu geringen Anteilen in die Ölphase übergeht.

Die EP 845503 beschreibt flüssige, mit Öl-mischbare Carotinoid-Zubereitungen in Form einer Wasser-in-Öl-Emulsion, worin die emulgierte wässrige Phase schutzkolloidstabilisierte Carotinoid-Partikel in dispergierter Form enthält. Als nachteilig erweist sich, dass das Astaxanthin beim Verdünnen der Formulierung mit für Nahrungsmittel geeigneten Ölen nur teilweise in die Ölphase übergeht.

Die WO 03/102116 beschreibt ölige Lösungen von Carotinoiden wie Astaxanthin. Ihre Herstellung erfolgt durch Lösen des Carotinoids in einem organischen Lösungsmittel wie N-Methylpyrrolidon in Anwesenheit eines lipophilen Dispergiermittels und Entfernen des Lösungsmittels. Das erhaltene Pulver wird dann in einem Öl, beispielsweise Fischöl, gelöst. Dieses Verfahren ist vergleichsweise aufwändig und bedarf größerer Mengen an Schutzkolloiden.

Die WO 2006/125591 beschreibt die Herstellung von öligen Carotinoid-Lösungen, beispielsweise Lösungen des Astaxanthins, wobei man zunächst eine Suspension des Carotinoids in der Ölphase bereitstellt, die Suspension für eine kurze Zeit auf hohe Temperaturen, z. B. im Bereich von 100 bis 230 °C, erhitzt, um das Carotinoid in Lösung zu bringen, und die heiße Lösung mit einem kalten Öl vermischt. Zur Herstellung von öligen Lösungen des Astaxanthins werden regelmäßig Temperaturen oberhalb 160 °C angewendet. Dieses Verfahren ist mit einer Reihe von Nachteilen verbunden. Zum einen führt das Erhitzen aufgrund der chemischen Labilität von ß-Carotinoiden unter anderem zu einer unerwünschten cis-trans-Isomerisierung der exocyclischen Doppelbindungen und damit zu einem Aktivitätsverlust. Zudem erweist sich die Bereitstellung öliger Suspensionen des Astaxanthins durch Vermahlen von Astaxanthin in Öl als problematisch.

Der vorliegenden Erfindung liegt die Aufgabe zu Grunde, geeignete Formulierungen von Astaxanthin oder einem Astaxanthin-Derivat bereitzustellen, die es erlauben, Astaxanthin oder das Astaxanthin-Derivat in ein für Nahrungsmittel geeignetes Öl einzuarbeiten, ohne dass es der Anwendung von Temperaturen oberhalb 100 °C bedarf. Dabei sollte das Astaxanthin-Derivat weitgehend oder vollständig in die Ölphase übergehen. Die Formulierungen sollten einfach herzustellen sein, und das darin enthaltene Astaxanthin sollte einen hohen Gehalt an all-trans Isomer aufweisen.

Die ältere internationale Patentanmeldung PCT/EP2008/061384 beschreibt ein Verfahren zur Herstellung von öligen Lösungen des Astaxanthins in für Nahrungsmittel geeigneten Ölen, bei denen man bestimmte Astaxanthin-Derivate der im Folgenden angegebenen Formel I zunächst in eine Suspension in einem essbaren Öl überführt, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen. Diese Suspensionen sind typischerweise bei Temperaturen von 25 °C flüssig und können in einfacher Weise mit flüssigen, für Nahrungsmittel geeigneten Ölen verdünnt werden, wobei sich die verwendeten Astaxanthin-Derivate aufgrund ihrer vorteilhaften Lösungskinetik und der geringen Partikelgröße rasch in dem zur Verdünnung eingesetzten Öl auflösen, ohne dass es der Anwendung von Temperaturen oberhalb 100 °C und/oder größerer Mengen an Emulgatoren bedarf.

Astaxanthin-Derivate der im Folgender angegebenen Formel I sind außerdem in WO 03/066583 offenbart, insbesondere in Pulverform.

Es wurde nun überraschenderweise gefunden, dass Astaxanthin-Derivate, die zu wenigstens 70 Gew.-% aus einer Verbindung der im Folgenden definierten Formel I bestehen, anders als Astaxanthin, besonders rasch in die Ölphase übergehen, wenn sie als eine Zusammensetzung formuliert werden, die eine kontinuierliche Ölphase wenigstens eines für Nahrungsmittel geeigneten Öls und eine in der Ölphase emulgierte wässrige Phase, in der Astaxanthin-Derivat in Form dispergierter, durch wenigstens ein Schutzkolloid stabilisierter Partikel vorliegt, umfasst.

Dementsprechend betrifft die vorliegende Erfindung Formulierungen eines Astaxanthin-Derivats, umfassend eine kontinuierliche Ölphase wenigstens eines für Nahrungsmittel geeigneten Öls und eine in der Ölphase emulgierte wässrige Phase, in der das Astaxanthin-Derivat in Form dispergierter, durch wenigstens ein Schutzkolloid stabilisierter Partikel vorliegt, wobei das Astaxanthin-Derivat zu wenigstens 70 Gew.-%, vorzugsweise wenigstens 80 Gew.-%, insbesondere wenigstens 90 Gew.-% und speziell wenigstens 96 Gew.-% aus einer Verbindung der im Folgenden definierten Formel I besteht:

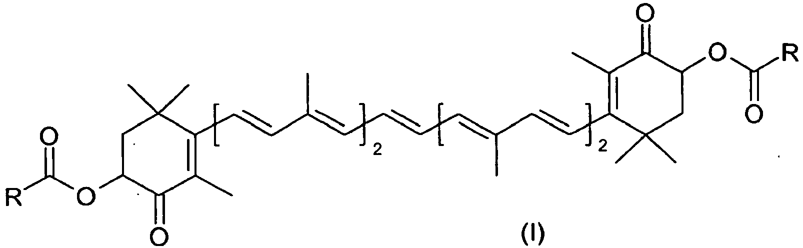

In Formel I steht R für einen Rest der Formel 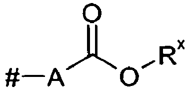 worin # die Anknüpfung an die Carbonylgruppe bedeutet, A für -CH₂CH₂- oder für -CH=CH- steht und R^{x} für C₁-C₄ Alkyl steht.

Die Erfindung ist mit einer Reihe von Vorteilen verbunden. Zum einen lassen sich unter Verwendung der erfindungsgemäßen Formulierungen in einfacher Weise Lösungen von Astaxanthin-Derivaten in für Nahrungsmittel geeigneten Ölen durch Einarbeiten der erfindungsgemäßen Zusammensetzung in das für Nahrungsmittel geeignete Öl herstellen. Anders als in dem in WO 2006/125591 beschriebenen Verfahren bedarf es hierzu grundsätzlich nicht der Anwendung von Temperaturen oberhalb 100 °C. Vielmehr kann man unter Verwendung der erfindungsgemäßen Formulierungen verdünnte Lösungen des Astaxanthin-Derivats in dem flüssigen Öl bei Temperaturen von unterhalb 100 °C, insbesondere maximal 90 °C oder gar maximal 80 °C, z. B. bei Temperaturen im Bereich von 20 bis 90 °C, insbesondere 30 bis 85 °C herstellen. Aufgrund der niedrigen Temperaturen zeichnen sich die auf diese Weise erhältlichen Lösungen durch einen sehr hohen Anteil an all-trans-Isomeren aus, der in der Regel oberhalb 70 %, häufig oberhalb 80 %, insbesondere oberhalb 90 %, bezogen auf das in der Formulierung enthaltene Astaxanthin-Derivat liegt. Ferner lassen sich unter Verwendung von Astaxanthin-Derivaten, die zu wenigstens 70 Gew.-% aus wenigstens einer Verbindungen der Formel I bestehen, sehr viel einfacher die erfindungsgemäßen Formulierungen herstellen als bei Verwendung des Astaxanthins selber oder anderer, davon verschiedener Astaxanthin-Derivate wie Astaxanthindiacetat oder Astaxanthindisuccinat.

In Formel I steht C₁-C₄-Alkyl für einen linearen oder verzweigten, gesättigten Kohlenwasserstoffrest mit 1 bis 4 C-Atomen wie Methyl, Ethyl, n-Propyl, 1-Methylethyl, Butyl, 1-Methylpropyl, 2-Methylpropyl oder 1,1-Dimethylethyl.

Die beiden Reste R in Formel I können gleich oder verschieden sein und sind vorzugsweise identisch.

Vorzugsweise steht A für 1,2-Ethandiyl. R^{x} steht vorzugsweise für Methyl oder Ethyl.

Verbindungen der Formel I, worin A im Rest R für 1,2-Ethandiyl steht, werden im Folgenden auch als Dialkyldisuccinate bezeichnet. Hierunter besonders bevorzugt ist das Dimethylsuccinat.

Die Verbindungen der Formel I können, da die die Gruppe O-C(O)R tragenden Kohlenstoffatome Asymmetriezentren sind, diastereomere und enantiomere Formen bilden, nämlich die 3R,3'S-Form, die 3S,3'R-Form, die 3R,3'R-Form und die 3S,3'S-Form. Sofern die Reste R in Formel I gleich sind, sind die 3R,3'S-Form und die 3S,3R-Form identisch und achiral (meso-Form), und die 3S,3'S-Form und die 3R,3'R-Form bilden ein Enantiomerenpaar. Für die Erfindung können sowohl die reinen Enantiomere und Diasteromere sowie Gemische der vorgenannten Diastereomere, z. B. ein racemisches Gemisch der 3S,3'S-Form und der 3R,3'R-Form als auch ein Gemisch der meso-Form mit der 3S,3'S-Form und/oder der 3R,3'R-Form oder ein Gemisch der meso-Form und dem Racemat der 3S,3'S-Form und der 3R,3'R-Form eingesetzt werden. In einer bevorzugten Ausgestaltung der Erfindung wird die Verbindung der Formel I in der 3S,3'S-Form oder einer Mischung der 3S,3'S-Form mit einer oder weiteren Formen der Verbindung der Formel I eingesetzt, worin die 3S,3'S-Form wenigstens 80 Gew.-%, insbesondere wenigstens 90 Gew.-%, bezogen auf die Gesamtmenge der Verbindung der Formel I ausmacht. In einer weiteren bevorzugten Ausgestaltung der Erfindung wird die Verbindung der Formel I in der 3R,3'R-Form oder einer Mischung der 3R,3'R-Form mit einer oder weiteren Formen der Verbindung der Formel I eingesetzt, worin die 3R,3'R-Form wenigstens 80 Gew.-%, insbesondere wenigstens 90 Gew.-%, bezogen auf die Gesamtmenge der Verbindung der Formel I ausmacht.

In den erfindungsgemäßen Formulierungen weist das Astaxanthin-Derivat üblicherweise eine Reinheit, bezogen auf das in Formel I gezeigten all-trans-Isomer, von wenigstens 70 Gew.-%, häufig wenigstens 80 Gew.-%, vorzugsweise wenigstens 90 Gew.-% und insbesondere wenigstens 96 Gew.-% auf. Neben dem all-trans-Isomeren kann das Astaxanthin-Derivat auch Anteile an Astaxanthin-Verbindungen der Formel I enthalten, in denen eine oder mehrere Doppelbindungen der in Formel I dargestellten all-trans-Isomere cis-Konfiguration aufweisen. Die Gesamtmenge an all-trans-Isomeren der Formel I und cis-Isomeren der Formel I beträgt in der Regel wenigstens 80 Gew.-% und häufig wenigstens 90 Gew.-%, bezogen auf das in der Zusammensetzung enthaltene Astaxanthin-Derivat. Neben dem all-trans-Isomeren der Formel I und etwaigem cis-Isomeren kann das Astaxanthin-Derivat auch weitere Carotinoide umfassen, wobei der Anteil dieser Verunreinigungen in der Regel 30 Gew.-%, häufig 20 Gew.-%, insbesondere 10 Gew.-%, besonders bevorzugt 4 Gew.-%, bezogen auf die Gesamtmenge des Astaxanthin-Derivats nicht überschreitet. Bei diesen Verunreinigungen handelt es sich in erster Linie um Halbester des Astaxanthins, d. h. um Verbindungen der im Folgenden gezeigten Formel II 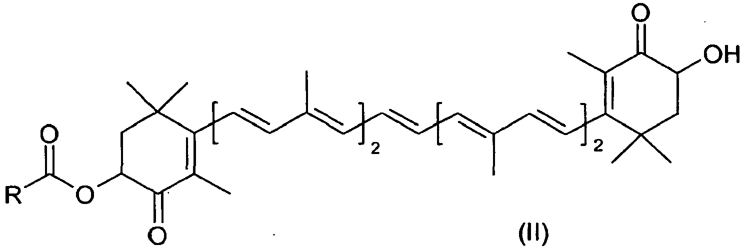 worin R die zuvor genannten Bedeutungen hat bzw. um cis-Isomere davon, sowie um Astaxanthin selber, Adonirubin und/oder Semi-Astacin.

Insbesondere enthält die erfindungsgemäße Zusammensetzung ein Astaxanthin-Derivat, das die an ein für Nahrungsmittel (d. h. für Lebens- und/oder Futtermittel) zugelassenes Astaxanthin gestellten Anforderungen erfüllt, d. h. das weniger als 4 Gew.-% Carotinoide, die von Astaxanthin und seinen Derivaten verschieden sind, enthält und das einen Schwermetallgehalt von höchstens 10 ppm aufweist, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat in der Zusammensetzung, aus einer Astaxanthin-Verbindung der Formel I besteht, wobei das in der Zusammensetzung enthaltene Astaxanthin-Derivat zu wenigstens 70 Gew.-%, häufig wenigstens 80 Gew.-%, vorzugsweise wenigstens 90 Gew.-% und insbesondere wenigstens 95 Gew.-% aus dem all-trans-Isomeren besteht.

Bei den erfindungsgemäßen Formulierungen handelt es sich um Wasser-in-Öl-Emulsionen, d. h. um die Formulierungen, die eine kontinuierliche Öl-Phase und eine in der kontinuierlichen Phase emulgierte wässrige Phase umfassen. Die Tröpfchen der wässrigen Phase wiederum enthalten das Astaxanthin-Derivat in Form dispergierter, durch wenigstens ein Schutzkolloid stabilisierter Partikel.

Die kontinuierliche Phase wird von einem für Nahrungsmittel geeigneten Öl gebildet, das gegebenenfalls weitere Bestandteile wie Emulgatoren und/oder Antioxidationsmittel in gelöster Form enthalten kann. Das Öl kann synthetischer, mineralischer, pflanzlicher oder tierischer Herkunft sein. Beispiel sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Tricyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl.

Bevorzugt sind pflanzliche Öle und Öle tierischen Ursprungs, die bei 40 °C, insbesondere bei 30 °C flüssig sind, insbesondere Pflanzenöle wie Sojabohnenöl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, MCT-Öle, weiterhin Fischöle, sowie Mischungen dieser Öle.

In einer bevorzugten Ausführungsform der Erfindung umfasst das Öl, welches die kontinuierliche Phase der Formulierung bildet, zu wenigstens 50 Gew.-% und insbesondere zu wenigstens 80 Gew.-%, bezogen auf die Gesamtmenge des Öls, wenigstens ein unter Maisöl, Sonnenblumenöl, Sojabohnenöl und Fischöl ausgewähltes essbares Öl.

Bei den Ölen kann es sich um Raffinatöle oder Rohöle handeln, die noch ursprungsspezifische Verunreinigungen wie Proteine, Phosphat, (Erd)alkalimetallsalze und dergleichen in üblichen Mengen enthalten.

Die Menge an Öl beträgt in der Regel 30 bis 90 Gew.-%, insbesondere 50 bis 80 Gew.-%, bezogen auf die Gesamtmasse der Formulierung.

Die erfindungsgemäße Formulierung enthält weiterhin Wasser, welches zusammen mit dem Astaxanthin-Derivat und dem Schutzkolloid und gegebenenfalls weiteren Bestandteile die disperse Phase, d. h. die emulgierte wässrige Phase bildet. Die Tröpfchen der emulgierten wässrigen Phase weisen typischerweise einen volumenmittleren Partikeldurchmesser unterhalb 100 µm, typischerweise von maximal 10 µm, z. B. im Bereich von 0,5 bis 10 µm, auf, wie er durch Lichtstreuung bestimmt werden kann.

Die Menge an Wasser beträgt in der Regel 5 bis 40 Gew.-%, insbesondere 8 bis 30 Gew.-%, bezogen auf das Gesamtgewicht der Formulierung..

Neben Wasser enthält die wässrige Phase das durch wenigstens ein Schutzkolloid stabilisierte Astaxanthin-Derivat in Partikelform. Die Partikel des Astaxanthin-Derivats weisen typischerweise mittlere Partikeldurchmesser (Gewichtsmittel, bestimmt durch Lichtstreuung) von maximal 2000 nm, z. B. im Bereich von 10 bis 2000 nm, auf. Insbesondere liegt der gewichtsmittlere Partikeldurchmesser im Bereich von 50 nm bis 1500 nm und speziell im Bereich von 80 nm bis 1000 nm.

Die Menge an Astaxanthin-Derivat beträgt in der Regel 0,1 bis 10 Gew.-%, insbesondere 0,5 bis 5 Gew.-%, bezogen auf das Gesamtgewicht der Formulierung. Bezogen auf die Menge an Wasser beträgt die Menge an Astaxanthin-Derivat in der Regel 1 bis 30 Gew.-%, insbesondere 2 bis 20 Gew.-%.

Weiterhin enthält die erfindungsgemäße Formulierung wenigstens ein Schutzkolloid zur Stabilisierung der Partikel des Astaxanthin-Derivats in der wässrigen Phase. Bei den Schutzkolloiden handelt es sich um in Wasser lösliche oder quellbare polymere Substanzen mit oberflächenaktiver Wirkung. Die Schutzkolloide bewirken eine Stabilisierung der Astaxanthinpartikel und verhindern auf diese Weise ihre Agglomeration.

Die Menge an Schutzkolloid beträgt in der Regel 1 bis 20 Gew.-%, insbesondere 2 bis 15 Gew.-%, bezogen auf das Gesamtgewicht der Formulierung.

Das Molekulargewicht (Gewichtsmittel) des Schutzkolloids liegt typischerweise im Bereich von 5000 bis 500 000 Dalton, insbesondere im Bereich von 10 000 bis 350 000 Dalton und speziell im Bereich von 15 000 bis 250 000 Dalton.

Das Gewichtsverhältnis von Schutzkolloid zu Astaxanthin-Derivat liegt typischerweise im Bereich von 50 : 1 bis 1 : 5, insbesondere im Bereich von 20 : 1 bis 1 : 2 und speziell im Bereich von 10 : 1 bis 1 : 1.

Geeignete Schutzkolloide sind ausgewählt ist unter
1) proteinischen Schutzkolloiden,
2) Ligninsulfonsäuren, deren Salzen, insbesondere deren Natriumsalzen und Kaliumsalzen,
3) Oligo- und Polysacchariden, einschließlich modifizierter Polysaccharide, wie beispielsweise Dextrine, Cellulose und Cellulosederivate wie Methylcellulose, Carboxymethylcellulose und deren Salze, Hydroxyethylcellulose, Hydroxypropylcellulose und Hydroxypropylmethylcellulose, Gummi-Arabicum, Pektine,
4) Polyvinylalkohol, einschließlich partiell verseiftem Polyvinylalkohol,
5) Polyvinylpyrrolidon und
Gemischen der vorgenannten Schutzkolloide.

Bei den proteinischen Schutzkolloiden der Gruppe 1) handelt es sich um eine in Wasser lösliche oder quellbare polymere Substanz, die aus Aminosäuren aufgebaut ist und gegebenenfalls glykosidische Bestandteile aufweist. Das proteinische Schutzkolloid ist in der Regel pflanzlichen oder tierischen Ursprungs. Beispiele für proteinische Schutzkolloide der Gruppe 1) sind Caseine und Caseinate, einschließlich αₛ₁-, αₛ₂-, ß- und κ-Kasein und deren Gemische, Prolamine, d. h. Speicherproteine aus Getreidesamen wie Gliadin, Secalin, Avenalin, Hordein, Zein, Oryzin und Kafirin, Molkeproteine wie beta-Lactoglobulin, alpha-Lactalbumin, Serumalbumin, Proteosepepton, Immunoglobuline und deren Gemische, Gelatine wie Rinder-, Schweine- und Fischgelatine, insbesondere sauer abgebaute Gelatine wie Gelatine A100 und A200, alkalisch abgebaute Gelatine wie Gelatine B100 und B200 sowie enzymatisch abgebaute Gelatinetypen wie die unter den Handelsbezeichnungen Gelita® Collagel A, Gelita® Sol DA, Gelita® Sol PA und Gelita® Sol LDA (DGF Stoess) erhältlichen Produkte, weiterhin Sojaprotein und teilhydrolysiertes Sojaprotein sowie Lupin-Protein.

Unter Lupin-Protein versteht man aus Lupine gewonnene Pflanzenproteine.

Gemäß einer bevorzugten Ausführungsform enthält die Zusammensetzung wenigstens ein proteinisches Schutzkolloid der Gruppe 1), gegebenenfalls in Kombination mit einem Oligo- oder Polysacharid der Gruppe 3).

Das Molekulargewicht (Gewichtsmittel) des proteinischen Schutzkolloids liegt typischerweise im Bereich von 10 000 bis 250 000 Dalton, insbesondere im Bereich von 15 000 bis 200 000 Dalton und speziell im Bereich von 20 000 bis 180 000 Dalton.

Das Gewichtsverhältnis von proteinischem Schutzkolloid zu Astaxanthin-Derivat liegt typischerweise im Bereich von 50 : 1 bis 1 : 5, insbesondere im Bereich von 20 : 1 bis 1 : 2 und speziell im Bereich von 10 : 1 bis 1 : 1.

Bevorzugte Schutzkolloide sind Casein, Caseinate z. B. Na-Caseinat, Sojaprotein, teilhydrolysiertes Sojaprotein, Prolamin und Lupin-Protein.

In einer bevorzugten Ausführungsform der Erfindung umfasst das proteinische Schutzkolloid ein Sojaprotein oder ein Gemisch aus Sojaprotein und einem weiteren proteinischen Schutzkolloid, z. B. ein Gemisch aus Sojaprotein und einem Prolamin, ein Gemisch aus Sojaprotein und einem Lupin-Protein, ein Gemisch aus Sojaprotein und einem und Casein und/oder Caseinat, ein Gemisch aus Sojaprotein und Sojaproteinhydrolysat. Insbesondere bildet das Sojaprotein den Hauptbestandteil des proteinischen Schutzkolloids, der Anteil am Schutzkolloid beträgt wenigstens 50 Gew.-%, insbesondere wenigstens 80 Gew.-% oder wenigstens 90 Gew.-%.

In einer weiteren, ebenfalls bevorzugten Ausführungsform der Erfindung umfasst das proteinische Schutzkolloid ein teilhydrolysiertes Sojaprotein oder ein Gemisch aus teilhydrolysiertem Sojaprotein und einem weiteren proteinischen Schutzkolloid, z. B. ein Gemisch aus teilhydrolysiertem Sojaprotein und einem Prolamin, ein Gemisch aus teilhydrolysiertem Sojaprotein und einem Lupin-Protein, ein Gemisch aus teilhydrolysiertem Sojaprotein und einem und Casein und/oder Caseinat. Insbesondere bildet das teilhydrolysierte Sojaprotein den Hauptbestandteil des proteinischen Schutzkolloids, der Anteil am Schutzkolloid beträgt wenigstens 50 Gew.-%, insbesondere wenigstens 80 Gew.-% oder wenigstens 90 Gew.-%.

Unter einem teilhydrolysierten Sojaprotein versteht man ein Sojaprotein, das einem partiellen enzymatischen Abbau mit einer Protease unterworfen wurde. Der Abbaugrad DH eines solchen teilhydrolysierten Sojaproteins beträgt in der Regel wenigstens 1 %, insbesondere wenigstens 5 %, z. B. im Bereich von 1 bis 20 % und insbesondere im Bereich von vorzugsweise bevorzugt 5 bis 16 %. Unter dem Abbaugrad DH versteht man den Quotienten aus der Anzahl der hydrolysierten Peptidbindungen zur Gesamtzahl der Peptidbindungen im Ausgangsprotein. Der Abbaugrad kann gemäß der so genannten "pH-Stat-Methode" bestimmt werden, wie von C. F. Jacobsen et al. in "Methods of Biochemical Analysis", Vol. IV, S. 171-210, Interscience Publishers Inc., New York 1957, beschrieben.

Als Sojaproteine für die Herstellung der erfindungsgemäßen Formulierungen, aber auch zur Herstellung der teilhydrolysierten Sojaproteine werden üblicherweise handelsübliche Sojaprotein-Isolate und -Konzentrate mit Proteingehalten von in der Regel 70 bis 90 Gew.-% eingesetzt, wobei die restlichen 10 bis 30 Gew.-% mehr oder weniger undefinierte andere Pflanzenbestandteile darstellen.

In einer weiteren Ausführungsform der Erfindung umfasst das Schutzkolloid wenigstens ein Ligninsulfonat, insbesondere Na-Ligninsulfonat, gegebenenfalls in Kombination mit weiteren Schutzkolloiden, insbesondere solchen aus der Gruppe 3). Insbesondere bildet das Ligninsulfonat in dieser Ausführungsform den Hauptbestandteil des Schutzkolloids, der Anteil am Schutzkolloid beträgt wenigstens 50 Gew.-%, insbesondere wenigstens 80 Gew.-% oder wenigstens 90 Gew.-%.

Die Formulierung kann auch weitere Bestandteile enthalten. Hierzu zählen insbesondere lipophile Dispergiermittel (Emulgatoren), die zur Stabilisierung der dispersen wässrigen Phase in der kontinuierlichen Ölphase geeignet sind. Typische lipophile Dispergiermittel sind Fettsäureester, insbesondere C₁₀-C₂₈-Fettsäureester der Ascorbinsäure wie Ascorbyloleat, Ascorbylstearat und Ascorbylpalmitat, Mono- und Diglyceride von Fettsäuren, insbesondere von C₁₀-C₂₈-Fettsäuren wie Glycerolmonostearat, Polyglycerin-Fettsäureester, Polyglycerin-C₁₀-C₂₈-Fettsäureester wie Polyglycerin-3-polyrizinoleat (PGPR90), Sorbitanfettsäureester, insbesondere Sorbitan-C₁₀-C₂₈-fettsäureester wie z. B. Mono- und Di-C₁₀-C₂₈-fettsäureester des Sorbitans wie Sorbitanmonolaurat, Sorbitanmonooleat und Sorbitanmonostearat (SPAN60), ethoxilierte Sorbitanfettsäureester, insbesondere ethoxilierte Sorbitan-C₁₀-C₂₈-fettsäureester wie PEG(20)-Sorbitolmonooleat, Monoester der Milchsäure mit gesättigten C₁₀-C₂₄-Fettsäuren, Zuckerester von gesättigten C₁₆-C₂₈-Fettsäuren, Propylenglycol-Fettsäureester, insbesondere Propylenglycol-C₁₀-C₂₈-Fettsäureester sowie Phospholipide wie Lecithin. Bevorzugte lipophile Dispergiermittel weisen einen HLB-Wert von maximal 10, z. B. im Bereich von 1,5 bis 10, insbesondere im Bereich von 2 bis 8 auf. Bezüglich des HLB-Wertes wird auf H. Mollet, A. Grubbemann, Formulation Technology, Wiley-VCH Verlag, Weinheim 2001, S. 69 bis 76, verwiesen.

Die Emulgatoren sind in der erfindungsgemäßen Formulierung typischerweise in Mengen von 1 bis 25 Gew.-%, insbesondere 2 bis 20 Gew.-%, bezogen auf das Gesamtgewicht der Formulierung, enthalten. Die Emulgatoren werden üblicherweise in Mengen von 0,1 bis 10 Gew.-Teilen, bezogen auf 1 Gew.-Teil des Astaxanthin-Derivats eingesetzt. Bezogen auf Wassermenge beträgt die Menge an lipophilem Dispergiermittel typischerweise 0,05 bis 1 Gew.-Teil, insbesondere 0,1 bis 0,8 Gew.-Teile, bezogen auf 1 Gew.-Teil Wasser.

Zu den weiteren Bestandteilen zählen auch Antioxidantien, Konservierungsmittel und so genannte Weichmacher.

Beispiele für Antioxidantien sind insbesondere Tocopherole wie α-Tocopherol, α-Tocopherolpalmitat, α-Tocopherolacetat, tert.-Butylhydroxytoluol, tert.-Butylhydroxyanisol, Ascorbinsäure, deren Salze und Ester wie z. B. Natriumascorbat, Calciumascorbat, Ascorbylphosphatester, Fettsäureester der Ascorbinsäure wie Ascorbylstearat und Ascorbylpalmitat und Ethoxyquin. Die Antioxidantien sind, sofern erwünscht, in der erfindungsgemäßen Formulierung typischerweise in Mengen von 0,01 bis 10 Gew.-%, häufig 0,1 bis 5 Gew.-%, bezogen auf das Gesamtgewicht der Formulierung, enthalten.

Beispiele für geeignete Konservierungsmittel sind Benzoesäure und ihre Salze, insbesondere ihre Natrium-, Kalium- und Calciumsalze, 4-Hydroxybenzoesäure (PHB) und ihre Salze, insbesondere ihre Natrium-, Kalium- und Calciumsalze, und ihre Alkylester (PHB-Alkylester), insbesondere ihre Methyl-, Ethyl- und Propylester und die Salze der PHB-Alkylester wie das Natriumsalz des PHB-Methylesters, das Natriumsalz des PHB-Ethylesters und das Natriumsalz des PHB-Propylesters, Sorbinsäure und ihre Salze, insbesondere ihre Natrium-, Kalium- und Calciumsalze, Propionsäure und ihre Salze wie insbesondere ihre Natrium-, Kalium- und Calciumsalze, Borsäure und Milchsäure. Konservierungsmittel sind, sofern erwünscht, in der erfindungsgemäßen Formulierung typischerweise in Mengen von 0,001 bis 5 Gew.-%, häufig 0,01 bis 1 Gew.-%, bezogen auf das Gesamtgewicht der Formulierung, enthalten.

Als Weichmacher kommen insbesondere Mono-, Di- und Oligosaccharide, insbesondere solche mit 3 bis 30 und speziell 5 bis 20 Monosaccharideinheiten, sowie Zuckeralkohole in Betracht, z. B. Saccharose, Glucose, Glucosesirup, Dextrine, insbesondere solche mit 5 bis 20 Glukose-Einheiten (z. B. solche, die unter der Bezeichnung "Glucidex" von der Fa. Rouquette vertrieben werden), Lactose, Invertzucker, Sorbit, Mannit, Glycerin und Mischungen der vorgenannten Substanzen. Bevorzugte Weichmacher sind Mono- und Disaccharide, die vorgenannten Dextrine mit 5 bis 20 Glukose-Einheiten sowie Sorbit und Mannit. In einer bevorzugen Ausführungsform der Erfindung enthält die Zusammensetzung wenigstens einen Weichmacher. Die Weichmacher sind in der Formulierung vorzugsweise in einer Menge von 1 bis 25 Gew.-%, insbesondere 2 bis 20 Gew.-%, bezogen auf das Gesamtgewicht der Formulierung, oder in einer Menge von 5 bis 70 Gew.-%, insbesondere 10 bis 60 Gew.-% bezogen auf die Gesamtmenge an Weichmacher, Schutzkolloid und Astaxanthin-Derivat, enthalten. Die Weichmacher bewirken in Kombination mit dem Schutzkolloid eine zusätzliche Stabilisierung der Partikel des Astaxanthin-Derivats.

Die Herstellung der erfindungsgemäßen Zusammensetzungen wird im Folgenden näher erläutert.

Die erfindungsgemäßen Zusammensetzungen sind in der Regel durch ein Verfahren erhältlich, welches die folgenden Schritte umfasst:
a) Bereitstellung einer wässrigen Dispersion des Astaxanthin-Derivats, die wenigstens ein Schutzkolloid enthält, wobei das Astaxanthin-Derivat zu wenigstens 70 Gew.-%, insbesondere wenigstens 80 Gew.-5, besonders bevorzugt wenigstens 90 Gew.-% oder wenigstens 96 Gew.-% aus der Verbindung der Formel I besteht; und
b) Emulgieren der wässrigen Dispersion des Astaxanthin-Derivats in wenigstens einem für Nahrungsmittel geeigneten Öl.

Das Verfahren kann in Analogie zu dem in EP 845503 beschriebenen Verfahren durchgeführt werden. Die Bereitstellung wässriger Dispersionen von Carotinoiden wie Astaxanthin, die ein Schutzkolloid enthalten, ist ebenfalls bekannt, z. B. aus DE 2534091, EP 065193, EP 278284, WO 00/6665, EP 1213013, EP 1219292 und EP 1228705, und kann in analoger Weise auf die Herstellung einer wässrigen Schutzkolloid-haltigen Dispersion des Astaxanthin-Derivats, das zu wenigstens 70 Gew.-%, insbesondere wenigstens 80 Gew.-%, besonders bevorzugt wenigstens 90 Gew.-% oder wenigstens 96 Gew.-% aus der Verbindung der Formel I besteht, übertragen werden.

Gegenstand der Erfindung ist daher auch ein derartiges Verfahren.

In der Regel erfolgt die Bereitstellung der wässrigen Dispersion des Astaxanthin-Derivats dergestalt, dass die Partikelgröße der dispergierten Partikel in der Regel einen gewichtsmittleren bzw. volumenmittleren Partikeldurchmesser von nicht mehr als 5 µm, häufig nicht mehr als 3 µm und insbesondere nicht mehr als 2 µm aufweisen, z. B. im Bereich von 10 nm bis 5 µm, häufig im Bereich von 20 nm bis 3 µm und insbesondere im Bereich von 50 nm bis 2 µm.

Gemäß einer ersten Ausführungsform des erfindungsgemäßen Verfahrens wird man ein festes Astaxanthin-Derivat, das in der Regel kristallin ist, in einer wässrigen Lösung des Schutzkolloids dispergieren und auf die gewünschte Partikelgröße vermahlen. Auf diese Weise werden wässrige Suspensionen des Astaxanthin-Derivats erhalten, worin die Partikel eine mittlere Teilchengröße (Volumenmittel) im Bereich von 0,1 bis 5 µm, insbesondere im Bereich von 0,2 bis 3 µm oder im Bereich von 0,3 bis 2 µm aufweisen (bestimmt durch Lichtstreuung).

Hierzu wird man zunächst eine grobteilige Suspension des Astaxanthin-Derivats, das vorzugsweise kristallin oder überwiegend kristallin ist (Kristallinitätsgrad > 80 %), in einer wässrigen Lösung des Schutzkolloids bereitstellen. Die wässrige Lösung des Schutzkolloids kann weitere, insbesondere wasserlösliche Bestandteile wie wasserlösliche Antioxidantien und Weichmacher enthalten.

Anschließend erfolgt die Mahlung auf die gewünschte Partikelgröße. Gegebenenfalls kann man während des Mahlens weitere Bestandteile der Formulierung zusetzen. Die Mahlung kann dabei in an sich bekannter Weise z. B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,1 bis 5 µm, bevorzugt 0,2 bis 3 µm, besonders bevorzugt 0,3 bis 2 µm, ganz besonders bevorzugt 0,3 bis 1,5 µm oder 0,3 bis 1 µm aufweisen. Der Begriff D[4,3] bezeichnet den volumengewichteten mittleren Durchmesser (Volumenmittel, siehe Handbuch zu Malvern Mastersizer S, Malvern Instruments Ltd., UK). Nähere Einzelheiten zur Mahlung und den dafür verwendeten Apparaturen finden sich u. a. in Ullmann's Encyclopedia of Industrial Chemistry, Sixth Edition, 1999, Electronic Release, Size Reduction, Kapitel 3.6.: Wet Grinding sowie in EP-A-0 498 824.

Gemäß einer zweiten Ausführungsform des erfindungsgemäßen Verfahrens wird man die Dispersion des Astaxanthin-Derivats dadurch bereitstellen, dass man eine Lösung des Astaxanthin-Derivats in einem geeigneten organischen Lösungsmittel oder in einer Mischung aus Wasser und einem geeigneten organischen Lösungsmittel in einer wässrigen Lösung des Schutzkolloids einbringt und anschließend das Lösungsmittel entfernt. Beim Einbringen der Lösung des Astaxanthin-Derivats in die wässrige Lösung des Schutzkolloids kommt es zu einer Fällung des Astaxanthin-Derivats und damit zur Ausbildung von Nanopartikeln des Astaxanthin-Derivats, worin das Astaxanthin-Derivat überwiegend oder vollständig amorph vorliegt. Auf diese Weise werden wässrige Dispersionen des Astaxanthin-Derivats erhalten, worin die Partikel eine mittlere Teilchengröße (Volumenmittel) im Bereich von 10 bis < 1000 nm, insbesondere im Bereich von 20 bis 500 nm und speziell im Bereich von 50 bis 300 nm, bestimmt durch Lichtstreuung, aufweisen

Die gemäß der zweiten Ausführungsform des erfindungsgemäßen Verfahrens zur Herstellung der Suspension verwendeten organischen Lösungsmittel sind in der Regel mit Wasser mischbar. Unter einem mit Wasser mischbaren organischen Lösungsmittel versteht man solche Lösungsmittel, die bei 25 °C zu wenigstens 10 Vol.-%, insbesondere zu wenigstens 50 Vol.-% oder vollständig mit Wasser mischbar sind. Bevorzug ist das in Schritt a) der zweiten Ausführungsform eingesetzte organische Lösungsmittel mit Wasser mischbar oder eine Mischung aus Wasser und einem mit Wasser mischbaren organischen Lösungsmittel. Bevorzugt sind solche organischen Lösungsmittel, die aus Kohlenstoff, Wasserstoff und Sauerstoff aufgebaut sind und mit Wasser mischbar sind. Vorzugsweise weist das organische Lösungsmittel bei Normaldruck einen Siedepunkt unterhalb 200 °C auf.

Bevorzugte organische, mit Wasser mischbare Lösungsmittel sind aus den Gruppen der C₁-C₄-Alkanole, C₂-C₄-Alkandiole, C₃-C₄-Ketone, Etheralkohole mit 3 bis 6 C-Atomen und cyclischen Ethern mit 4 bis 5 C-Atomen ausgewählt. Beispiele für bevorzugte, mit Wasser mischbare Lösungsmittel sind Methanol, Ethanol, n-Propanol, Isopropanol, Propandiol, Ethylenglykolmethylether, 1,2-Butandiol-1-methylether, 1,2-Propandiol-1-n-propylether, Tetrahydrofuran oder Aceton.

Vorzugsweise wird man bei der Herstellung gemäß der zweiten Ausführungsform so vorgehen, dass man die Lösung des Astaxanthin-Derivats in dem organischen Lösungsmittel oder einer Mischung aus organischem Lösungsmittel und Wasser, die eine Temperatur von wenigstens 30 °C, insbesondere wenigstens 50 °C, besonders bevorzugt wenigstens 100 °C, z. B. 30 bis 200 °C, insbesondere 50 bis 190 °C oder 80 bis 180 °C aufweist, in eine wässrige Lösung des Schutzkolloids einträgt und dadurch eine Fällung oder Emulgierung des Astaxanthin-Derivats bewirkt. Vorzugsweise weist die wässrige Lösung des Schutzkolloids eine Temperatur unterhalb der Temperatur der Lösung des Astaxanthin-Derivats auf und soll vorzugsweise eine Temperatur von 50 °C nicht überschreiten. Vorzugsweise wird die Temperatur und Menge der wässrigen Lösung des Schutzkolloids so gewählt, dass eine Mischungstemperatur unterhalb 100 °C, z. B. im Bereich von 20 bis 80 °C, resultiert.

Vorzugsweise wählt man die Bedingungen des Lösens so, dass die zum Lösen benötigte Zeit nicht mehr als 20 sec., insbesondere nicht mehr als 10 sec. und speziell nicht mehr als 2 sec. beträgt, z. B. 0,1 bis 20 sec. oder 0,1 bis 1 sec. Vorzugsweise erfolgt das Lösen unter Druck, z. B. bei einem Druck im Bereich von 10 bis 100 bar und insbesondere im Bereich von 20 bis 80 bar.

Die Konzentration an Astaxanthin-Derivat in der Lösung wird in der Regel im Bereich von 0,1 bis 10 Gew.-% liegen. Die Lösung des Astaxanthin-Derivats kann gegebenenfalls weitere Bestandteile der erfindungsgemäßen Zusammensetzung enthalten, die in dem Lösungsmittel bzw. in dem Lösungsmittel/Wassergemisch löslich sind. Hierzu zählen insbesondere die vorgenanten Antioxidantien, Emulgatoren und Öle, insbesondere eines der zuvor als bevorzugt angegebenen für Nahrungsmittel geeigneten Öle.

Die Konzentration an Schutzkolloid in der wässrigen Lösung wird in der Regel im Bereich von 0,1 bis 10 Gew.-% liegen. Die Lösung des Schutzkolloids kann gegebenenfalls weitere Bestandteile der erfindungsgemäßen Zusammensetzung enthalten, die in Wasser löslich oder suspendierbar sind. Hierzu zählen insbesondere die vorgenannten Weichmacher und Konservierungsmittel.

Die relativen Mengen an Lösung des Astaxanthin-Derivats und wässriger Lösung des Schutzkolloids werden in der Regel so gewählt, dass die relativen Mengenanteile der darin enthaltenen Bestandteile den relativen Mengenanteilen dieser Bestandteile in erfindungsgemäßen Formulierungen im Wesentlichen entsprechen.

Vorzugsweise führt man das Lösen und Einbringen in die Lösung des Schutzkolloids in Analogie zu der in EP 65193 beschriebenen Vorgehensweise durch. Hinsichtlich einer detaillierten Apparatebeschreibung wird auf die EP 65193 und die Beispiele dieser Anmeldung Bezug genommen.

Häufig wird man so vorgehen, dass man das Astaxanthin-Derivat, gegebenenfalls zusammen mit dem Antioxidationsmittel, gegebenenfalls dem Emulgator und gegebenenfalls einem für Nahrungsmittel geeigneten Öl in dem organischen Lösungsmittel oder einer Mischung aus organischem Lösungsmittel und Wasser unter den zuvor genannten Bedingungen in ein oder mehreren Stufen löst, vorzugsweise bei erhöhter Temperatur und erhöhtem Druck, löst und möglichst rasch in die wässrige Lösung des Schutzkolloids, die weitere Bestandteile wie Konservierungsmittel und Weichmacher enthalten kann, einträgt, wobei das Eintragen möglichst rasch erfolgt und geeignete Maßnahmen zur Homogenisierung der wässrigen Lösung des Schutzkolloids und der zugeführten Lösung des Astaxanthin-Derivats vorgesehen sind.

Insbesondere wird man so vorgehen, dass man das Astaxanthin-Derivat in dem organischen Lösungsmittel oder einer Mischung aus Wasser und organischem Lösungsmittel, gegebenenfalls zusammen mit den weiteren Bestandteilen der Lösung vermischt und kontinuierlich, gegebenenfalls unter Zuführung von weiterem Lösungsmittel, erhitzt, wobei man einen Volumenstrom der Lösung des Astaxanthin-Derivats erzeugt, den man mit einem Volumenstrom der Lösung des Schutzkolloids in einer geeigneten Mischvorrichtung, beispielsweise einem statischen oder dynamischen Mischer vermischt, wobei man die wässrige Suspension des Astaxanthin-Derivats, enthaltend das proteinische Schutzkolloid, erhält. Die Volumenströme werde dabei so gewählt, dass die Mengenanteile der darin enthaltenen Bestandteile den Mengenanteilen dieser Bestandteile in erfindungsgemäßer Zusammensetzung im Wesentlichen entsprechen.

Die auf diese Weise erhaltenen wässrigen Dispersionen des Astaxanthin-Derivats enthalten typischer 0,01 bis 5 Gew.-% des Astaxanthin-Derivats und 0,1 bis 10 Gew.-% des Schutzkolloids sowie gegebenenfalls 0,1 bis 10 Gew.-% des Weichmachers und gegebenenfalls weitere Bestandteile.

Vorzugsweise wird man die in Schritt a) erhaltene Suspension vor dem Emulgieren aufkonzentrieren, vorzugsweise auf einen Gehalt an Astaxanthin-Derivat im Bereich von 1 bis 20 Gew.-%, insbesondere 5 bis 15 Gew.-% und anschließend die Emulgierung durchführen.

Das Aufkonzentrieren kann in hierzu üblichen Vorrichtungen, beispielsweise Verdampferanordnungen wie Dünnfilmverdampfern und dergleichen, erfolgen.

Anschließend werden die so erhältlichen wässrigen Dispersionen des Astaxanthin-Derivats in dem für Nahrungsmittel geeigneten Öl, vorzugsweise in Gegenwart wenigstens eines für die Stabilisierung einer dispersen wässrigen Phase in einer hydrophoben Ölphase geeigneten lipophilen Dispergiermittels (Emulgator) emulgiert.

Vorzugsweise wird die Menge an wässriger Dispersion, bezogen auf das Gesamtgewicht der Formulierung, d. h. die Gesamtmenge an Öl, ggf. Emulgator und wässrige Phase, im Bereich von 9 bis 69 Gew.-%, insbesondere 18 bis 38 Gew.-% liegen.

Als Emulgatoren kommen alle der zuvor genannten lipophilen Dispergiermittel in Betracht. Bevorzugte Emulgatoren sind die vorgenannten Mono- und Diglyceride von Fettsäuren, insbesondere von C₁₀-C₂₈-Fettsäuren wie Glycerolmonostearat, Polyglycerin-Fettsäureester, Polyglycerin-C₁₀-C₂₈-Fettsäureester wie Polyglycerin-3-polyrizinoleat (PGPR90), Sorbitanfettsäureester, insbesondere Sorbitan-C₁₀-C₂₈-fettsäureester wie z. B. Mono- und Di-C₁₀-C₂₈-fettsäureester des Sorbitans wie Sorbitanmonolaurat, Sorbitanmonooleat und Sorbitanmonostearat (SPAN60), ethoxilierte Sorbitanfettsäureester, insbesondere ethoxilierte Sorbitan-C₁₀-C₂₈-fettsäureester wie PEG(20)-Sorbitolmonooleat und Propylenglycol-Fettsäureester, insbesondere Propylenglycol-C₁₀-C₂₈-Fettsäureester. Bevorzugte Emulgatoren weisen einen HLB-Wert von maximal 10, z. B. im Bereich von 1,5 bis 10, insbesondere im Bereich von 2 bis 8 auf.

Die Emulgatoren werden typischerweise in Mengen von 1 bis 25 Gew.-%, insbesondere 2 bis 20 Gew.-%, bezogen auf das Gesamtgewicht der Formulierung, verwendet. Bezogen auf wässrige Phase beträgt die Menge an Emulgator typischerweise 0,01 bis 0,8 Gew.-Teile, insbesondere 0,05 bis 0,5 Gew.-Teile, bezogen auf 1 Gew.-Teil wässrige Phase.

Die Emulgierung der wässrigen Dispersion kann in üblicher Weise erfolgen. In der Regel wird man die wässrige Dispersion zu dem Öl, das gegebenenfalls einen oder mehrere Emulgatoren enthält, geben, und anschließend unter Einwirkung von Scherkräften durchmischen, bis sich eine stabile Emulsion gebildet hat. Man kann das Emulgieren kontinuierlich oder diskontinuierlich durchführen. In der Regel erfolgt das Emulgieren bei Temperaturen im Bereich von 20 bis 80 °C, bevorzugt 40 bis 70 °C.

Für die physikalische Stabilität der erfindungsgemäßen Formulierung, wie etwa die Sedimentationsstabilität, hat es sich als vorteilhaft erwiesen, wenn eine sehr gute Feinverteilung der Wasserphase in der Ölphase durch Einwirkung starker Scherkräfte erzielt wird, so dass die mittleren Durchmesser (Volumenmittel) der wässrigen Phase nicht mehr als 10 µm und insbesondere nicht mehr als 5 µm oder 2 µm betragen. Dies gelingt beispielsweise durch Emulgierung mittels eines Rotor/Stator-Dispergator oder mit einem Hochdruckhomogenisator wie einem APV Gaulin bzw. mit einem Höchstdruckhomogenisator wie dem Microfluidizer im Druckbereich von 700 bis 1000 bar.

Alternativ kann man auch so vorgehen, dass man die in Schritt a) erhaltene wässrige Dispersion, die vorzugsweise in der oben beschriebenen Weise aufkonzentriert wurde, trocknet, gegebenenfalls unter Zusatz von Trocknungshilfsmitteln, und anschließend in Wasser auf die gewünschte Konzentration redispergiert.

Die auf diese Weise erhältlichen Pulver sind neu. Die auf diese Weise erhältlichen Pulver weisen in der Regel die folgende Zusammensetzung auf
i) ein Astaxanthin-Derivat, das zu wenigstens 70 Gew.-%, vorzugsweise wenigstens 80 Gew.-%, insbesondere wenigstens 90 Gew.-% und speziell wenigstens 96 Gew.-% aus einer Verbindung der Formel I besteht, in einer Menge von 1 bis 50 Gew.-%, insbesondere 2 bis 30 Gew.-% und speziell 50 bis 20 Gew.-%, bezogen auf das Gesamtgewicht des Pulvers,
ii) wenigstens einem Schutzkolloid wie zuvor definiert in einer Menge von wenigstens 10 bis 70 Gew.-%, insbesondere 15 bis 65 Gew.-% und speziell 20 bis 60 Gew.-%, bezogen auf das Gesamtgewicht des Pulvers, und
iii) gegebenenfalls einen oder mehrere, für Nahrungsmittel geeigneten Zusatzstoffen in einer Menge von bis zu 89 Gew.-%, insbesondere bis 83 Gew.-%, speziell bis 75 Gew.-%, z. B. 10 bis 89 Gew.-%, insbesondere 15 bis 83 Gew.-% und speziell 20 bis 75 Gew.%, bezogen auf das Gesamtgewicht der Zusammensetzung.

Bei den Schutzkolloiden handelt es sich um die vorgenannten Schutzkolloide, insbesondere um die in den bevorzugten Ausführungsformen genannten Schutzkolloide.

Bei den Zusatzstoffen handelt es sich insbesondere um Oxidationsstabilisatoren (Anti-oxidantien), Emulgatoren, Konservierungsmittel, Weichmacher, Filmbildner, Füllstoffe, Öle, Überzugsmittel und Puderungsmittel. Der Gesamtmenge dieser Bestandteile beträgt in den Pulvern maximal 89 Gew.-%, z. B. 10 bis 89 Gew.-%, bezogen auf das Gesamtgewicht des Pulvers, und wird insbesondere 83 Gew.-% und insbesondere 75 Gew.-%, bezogen auf das Gesamtgewicht des Pulvers nicht überschreiten und liegt dann vorzugsweise im Bereich von 15 bis 83 Gew.-%, insbesondere im Bereich von 20 bis 75 Gew.-%, bezogen auf das Gesamtgewicht des Pulvers.

Beispiele für Antioxidantien sind insbesondere Tocopherole wie α-Tocopherol, α-Tocopherolpalmitat, α-Tocopherolacetat, tert.-Butylhydroxytoluol, tert.-Butylhydroxyanisol, Ascorbinsäure, deren Salze und Ester wie z. B. Natriumascorbat, Calciumascorbat, Ascorbylphosphatester, Fettsäureester der Ascorbinsäure wie Ascorbylstearat und Ascorbylpalmitat und Ethoxyquin. Die Antioxidantien sind, sofern erwünscht, in dem Pulver typischerweise in Mengen von 0,1 bis 20 Gew.-%, häufig 0,5 bis 10 Gew.-%, bezogen auf das Gesamtgewicht des Pulvers, enthalten.

Beispiele für Emulgatoren sind insbesondere die vorgenannten lipophilen Dispergiermittel. Emulgatoren sind, sofern erwünscht, in dem Pulver typischerweise in Mengen von 0,1 bis 20, häufig 0,5 bis 10 Gew.-%, bezogen auf das Gesamtgewicht des Pulvers, vorzugsweise in einer Menge von 1 bis 30 Gew.-%, insbesondere in einer Menge von 2 bis 15 Gew.-%, bezogen auf das Astaxanthin-Derivat, enthalten.

Beispiele für geeignete Konservierungsmittel sind die zuvor genannten Konservierungsmittel. Konservierungsmittel sind, sofern erwünscht, in dem Pulver typischerweise in Mengen von 0,001 bis 10 Gew.-%, häufig 0,01 bis 5 Gew.-%, bezogen auf das Gesamtgewicht des Pulvers, enthalten.

Als Weichmacher kommen insbesondere die zuvor genannten Mono-, Di- und Oligosaccharide sowie Zuckeralkohole in Betracht. Die Weichmacher sind vorzugsweise in einer Menge von 5 bis 70 Gew.-%, insbesondere 10 bis 60 Gew.-% in dem Pulver enthalten. Die Weichmacher wirken in der Regel auch als Filmbildner.

Die vorstehend beschriebenen Pulver können auch Füllstoffe enthalten. Hierunter versteht man inerte, feste partikelförmige Substanzen, wie beispielsweise feinteilige Kieselsäure, wie pyrogene Kieselsäure oder Kieselgel (E 551), Titandioxid, Hydroxide wie Aluminiumhydroxid, Carbonate und Hydrogencarbonate wie Natriumcarbonat (E 500), Natriumhydrogencarbonat, Kaliumcarbonat (E 501), Magnesiumcarbonat (E 504) und Calciumcarbonat (E 170), Silikate wie Natriumsilikat (E 550), Magnesiumsilikat (E 553a), Talk (E 553b), Calciumsilikat (E 552), Zinksilikat (E 557), Aluminiumsilikate wie Natriumaluminiumsilikat (554), Kaliumaluminiumsilikat (E 555), Calciumaluminiumsilikat (E 556), Bentonit (E 558), Kaolin (E 559), Tricalciumphosphat, Natriumchlorid, inerte, für Nahrungsmittel geeignete feste organische Materialien in Pulverform, z. B. ggf. modifizierte Polysacharide wie Stärke, Cellulose und Methylcellulose. Der Anteil der Füllstoffe wird in der Regel 50 Gew.-%, insbesondere 30 Gew.-% des Pulvers nicht überschreiten. Bevorzugte Füllstoffe sind die vorgenannten Polysacharide.

Die vorstehend beschriebenen Pulver können auch Öle enthalten. Der Ölanteil liegt, sofern erwünscht, vorzugsweise im Bereich von 0,1 bis 10 Gew.-%, bezogen auf das Gesamtgewicht der Zusammensetzung. Beispiele für Öle sind insbesondere Fettsäureglyceride, die natürlichen, z. B. tierischen oder pflanzlichen, Ursprungs, synthetisch oder semisynthetisch sein können. Beispiele für geeignete Öle sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Tricyceride wie die Miglyol-Typen, weiterhin tierische Öle und Fette wie Fischöle einschließlich Makrelen-, Sprotten-, Thunfisch-, Heilbutt-, Kabeljau- und Lachsöl und Lanolin. Die Öle sind, sofern erwünscht, in dem Pulver typischerweise in Mengen von 0,5 bis 30 Gew.-%, häufig 1 bis 20 Gew.-%, bezogen auf das Gesamtgewicht des Pulvers, enthalten.

Beispiele für Überzugsmittel sind Maisstärke, Kieselsäure und Tricalciumphosphat. Ihre Menge beträgt, sofern erwünscht, typischerweise 0,01 bis 10 Gew.-%, bezogen auf das Gesamtgewicht des Pulvers.

Die vorstehend beschriebenen Pulver bilden beim Dispergieren in Wasser eine feinteilige Dispersion von Partikeln des Astaxanthin-Derivats, die durch das Schutzkolloid stabilisiert werden. Die Partikelgröße kann, in Abhängigkeit von der Herstellung der Pulver über weite Bereiche variieren, wobei die Partikel in der Regel einen gewichtsmittleren bzw. volumenmittleren Partikeldurchmesser von nicht mehr als 5 µm, häufig nicht mehr als 3 µm und insbesondere nicht mehr als 2 µm aufweisen, z. B. im Bereich von 10 nm bis 5 µm, häufig im Bereich von 20 nm bis 3 µm und insbesondere im Bereich von 50 nm bis 2 µm.

Die Herstellung der vorstehend beschriebenen Pulver kann, wie bereits erwähnt, durch übliche Trocknung der oben beschriebenen wässrigen Dispersionen des Astaxanthin-Derivats erfolgen. Die Trocknung kann in einer oder mehreren Stufen erfolgen. Vorzugsweise wird man zunächst die in Schritt a) erhaltene Dispersion des Astaxanthin-Derivats aufkonzentrieren, vorzugsweise auf einen Gehalt an Astaxanthin-Derivat im Bereich von 1 bis 20 Gew.-%, und anschließend den eigentlichen Trocknungsschritt durchführen.

Das Aufkonzentrieren kann in hierzu üblichen Vorrichtungen, beispielsweise Verdampferanordnungen wie Dünnfilmverdampfern und dergleichen, erfolgen.

Die Trocknung kann nach üblichen Verfahren zur Herstellung von Pulvern aus wässrigen Dispersionen erfolgen. Zu nennen sind beispielsweise die Sprühtrocknung, Sprühkühlung, Gefriertrocknung, Sprühformulierung oder Sprühgranulierung, wobei Sprühtrocknung und Sprühformulierung, d. h. Sprühtrocknung in Gegenwart eines im Wirbelbett fluidierten interten Pulvers, bevorzugt sind. Gegebenenfalls kann die Trocknung in Gegenwart eines Überzugsmaterials erfolgen. Als Überzugsmittel eignen sich u. a. Maisstärke, Kieselsäure oder auch Tricalciumphosphat.

Die erfindungsgemäßen Formulierungen lassen sich leicht in für Nahrungsmittel geeignete Öle einarbeiten und sind daher insbesondere geeignet zur Herstellung verdünnter Lösungen des Astaxanthin-Derivats in für Nahrungsmittel geeigneten Ölen. Dementsprechend betrifft die vorliegende Erfindung auch die Verwendung einer solchen Formulierung zur Herstellung von Lösungen des Astaxanthin-Derivats in einem für Nahrungsmittel geeigneten Öl. Dabei hat sich gezeigt, dass bei Einarbeitung der erfindungsgemäßen Formulierung in ein für Nahrungsmittel geeignetes Öl, der Übertritt des Astaxanthin-Derivats in die Ölphase sehr viel rascher und vollständiger erfolgt und auch bei niedrigeren Temperaturen stattfindet als bei vergleichbaren Formulierungen des Astaxanthins selber.

Die vorliegende Erfindung betrifft auch die Verwendung der erfindungsgemäßen Formulierungen zur Herstellung von Futtermittelzubereitungen, insbesondere von festen Futtermittelzubereitungen, speziell Futtermittelzubereitungen in Form von Pellets. Dementsprechend betrifft ein weiterer Gegenstand der Erfindung ein Verfahren zur Herstellung von Futtermittelzubereitungen, insbesondere von festen Futtermittelzubereitungen, speziell von Pellets, umfassend:
α) Bereitstellung einer erfindungsgemäßen Formulierung wie hier und in den Ansprüchen beschrieben;
ß) Einarbeiten der Formulierung in ein für Nahrungsmittel geeignetes, flüssiges Öl unter Erhalt einer verdünnten flüssigen Lösung des Astaxanthin-Derivats in dem flüssigen Öl; und
γ) Vermischen der in Schritt ß) erhaltenen flüssigen Lösung mit den Bestandteilen der Futtermittelzubereitung oder Imprägnieren einer festen Futtermittelzubereitung mit der in Schritt ß) erhaltenen flüssigen Lösung.

Das in Schritt ß) eingesetzte Öl kann synthetischer, mineralischer, pflanzlicher oder tierischer Herkunft sein. Beispiele sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkemöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/-Caprinsäure-Tricyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl.

Bevorzugt sind pflanzliche Öle und Öle tierischen Ursprungs, die bei 40 °C flüssig sind, insbesondere Pflanzenöle wie Sojabohnenöl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, MCT-Öle, weiterhin Fischöle, sowie Mischungen dieser Öle.

In einer bevorzugten Ausführungsform der Erfindung umfasst das in Schritt ß) eingesetzte Öl zu wenigstens 50 Gew.-% und insbesondere zu wenigstens 80 Gew.-%, bezogen auf die Gesamtmenge des in Schritt ß) eingesetzten essbaren Öls, wenigstens ein unter Maisöl, Sonnenblumenöl, Sojabohnenöl und Fischöl ausgewähltes essbares Öl.

Bei den in Schritt ß) eingesetzten Ölen kann es sich um Raffinatöle oder Rohöle handeln, die noch ursprungsspezifische Verunreinigungen wie Proteine, Phosphat, (Erd)alkalimetallsalze und dergleichen in üblichen Mengen enthalten. Die in Schritt ß) eingesetzten Öle können auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des in Schritt b) eingesetzten Öls, enthalten.

Das Einarbeiten der erfindungsgemäßen Formulierung in das für Nahrungsmittel geeignete Öl erfolgt typischerweise bei Temperaturen unterhalb 100 °C, vorzugsweise bei Temperaturen von nicht mehr als 95 °C und speziell nicht mehr als 90 °C und vorzugsweise bei Temperaturen im Bereich von 20 bis < 100 °C, insbesondere im Bereich von 25 bis 95 °C und speziell im Bereich von 30 bis 90 °C.

Gegebenenfalls wird man das Öl, in welches man die erfindungsgemäße Formulierung des Astaxanthin-Derivats einarbeitet, erwärmen. In der Regel liegt die Temperatur des erwärmten Öls vorzugsweise unterhalb 100 °C und beträgt insbesondere nicht mehr als 95 °C und speziell nicht mehr als 90 °C, und liegt vorzugsweise im Bereich von 25 bis < 100 °C, insbesondere im Bereich von 30 bis 95 °C und speziell im Bereich von 35 bis 90 °C.

Typischerweise wird man die relativen Mengen an Öl und erfindungsgemäßer Formulierung so wählen, dass der Gehalt an Astaxanthin-Derivat in der resultierenden Lösung im Bereich von 10 ppm (= 0,001 Gew.-%) bis etwa 1 Gew.-%, häufig im Bereich von 20 ppm bis 0,5 Gew.-%, insbesondere im Bereich von 50 ppm bis 0,2 Gew.-%, bezogen auf die Gesamtmenge an Öl plus Astaxanthin-Derivat liegt.

Beispielsweise kann man so vorgehen, dass man ein für Nahrungsmittel geeignetes Öl, das vorzugsweise einen Schmelzpunkt von nicht mehr als 40 °C aufweist, in einem geeigneten Gefäß vorlegt, gegebenenfalls auf die gewünschte Temperatur vorwärmt und hierzu die erfindungsgemäße Formulierung unter Durchmischen zugibt, wobei die Zugabe in einer Portion, in mehreren Portionen oder kontinuierlich erfolgen kann. Die zugegebene erfindungsgemäße Zusammensetzung kann auf die gewünschte Temperatur vorgewärmt werden, was jedoch grundsätzlich nicht erforderlich ist.

Die zum Erreichen des Lösens erforderliche Zeit hängt naturgemäß von der Temperatur, dem gewählten Öl und den apparativen Gegebenheiten ab und liegt typischerweise im Bereich von 1 min. bis 120 min. Der Fachmann kann die erforderliche Zeit durch Routineexperimente ermitteln.

Im Anschluss an das Lösen der erfindungsgemäßen Formulierung kann man die erhaltene ölige Lösung, sofern erforderlich, abkühlen, z. B. mittels geeigneter Wärmetauscher oder durch Verdünnen mit einem kalten essbaren Öl, das zu dem zum Lösen in Schritt ß) verwendeten Öl gleich oder verschieden sein kann.

Auf diese Weise erhält man stabile Lösungen von Astaxanthin-Derivaten in für Nahrungsmittel geeigneten Ölen. Diese Lösungen zeichnen sich dadurch aus, dass das in ihnen enthaltene Astaxanthin-Derivat einen hohen Anteil an all-trans-Isomeren aufweist, der in der Regel oberhalb 80 %, insbesondere oberhalb 90 % liegt.

Die so erhaltenen Lösungen enthalten neben dem Astaxanthin-Derivat die in der Formulierung enthaltenen Substanzen, die sich nicht notwendigerweise lösen müssen, sowie ggf. weitere Zusätze wie Oxidationsstabilisatoren und lipophile Dispergiermittel.

Es versteht sich von selber, dass die in Schritt ß) erhaltenen Lösungen geringe Mengen an Wasser aus dem zu ihrer Herstellung eingesetzten, für Nahrungsmittel geeigneten Öl enthalten können. Häufig jedoch beträgt der Wasseranteil nicht mehr als 10 Gew.-%, z. B. 0,1 bis 10 Gew.-%, häufig 0,5 bis 8 Gew.-%, und in einer speziellen Ausführungsform der Erfindung nicht mehr als 0,5 Gew.-%, bezogen auf das in der Lösung enthaltene Öls.

Die nach dem erfindungsgemäßen Verfahren erhältlichen Lösungen des Astaxanthin-Derivats sind lagerstabil und können vor ihrer weiteren Verwendung über längere Zeiträume aufbewahrt werden, ohne dass es in nennenswertem Umfang zu einem Aktivitätsverlust, z. B. durch Isomerisierung und/oder oxidativen Abbau, kommt. Insbesondere zeichnen sie sich durch einen hohen Anteil an all-trans-Isomeren des eingesetzten Astaxanthin-Derivats aus und durch einen vergleichsweise geringen Anteil an Abbauprodukten des Astaxanthin-Derivats.

Das erfindungsgemäße Verfahren wird häufig direkt in die Prozesse zur Weiterverarbeitung der Lösungen des Astaxanthin-Derivats integriert werden.

Die nach dem erfindungsgemäßen Verfahren erhältlichen Lösungen des Astaxanthin-Derivats eignen sich in vorteilhafter Weise als Zusatz zu Tierfuttermitteln, Nahrungsmitteln für die Humanernährung, Nahrungsergänzungsmitteln, pharmazeutischen Mitteln oder kosmetischen Mitteln. Bevorzugt lassen sich die Lösungen als Futtermittelzusatz in der Tieremährung einsetzen, beispielsweise bei der Futtermittelherstellung durch Einmischen in einen Futtermittelteig vor oder während der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als so genannte "post-pelleting liquid application". Vorzugsweise belädt man die Futtermittelpellets mit den Formulierungen unter vermindertem Druck.

Bevorzugte Ausführungsformen betreffen Tierfuttermittel, insbesondere Fischfuttermittel, die das bei 30°C flüssige Öl, das feste Verdünnungsmittel und das Astaxanthin-Derivat umfassen. Derartige Mittel enthalten das in der Lösung enthaltene Astaxanthin-Derivat typischerweise in einer Menge von 10 bis 100 ppm, bezogen auf das Gesamtgewicht des Mittels, wobei das Astaxanthin-Derivat in der Regel zu mehr als 70 %, insbesondere zu wenigstens 80 % und speziell zu wenigstens 90 % eine all-trans-Konfiguration aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform dieser Mittel betrifft Futtermittelpellets, speziell Futtermittelpellets für Fischfutter, die mit der erfindungsgemäß erhältlichen Lösung des Astaxanthin-Derivats beladen sind. Derartige Pellets enthalten das in der Lösung enthaltene Astaxanthin-Derivat typischerweise in einer Menge von 10 bis 100 ppm, bezogen auf das Gesamtgewicht des Futtermittels. Die Herstellung solcher Pellets erfolgt in der Regel durch Besprühen konventioneller Pellets mit einer erfindungsgemäß erhältlichen Lösung des Astaxanthin-Derivats, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenfalls kann man erneut Vakuum anlegen und erneut die Lösung des Astaxanthin-Derivats oder ein für Nahrungsmittel geeignetes, flüssiges Öl in der zuvor beschriebenen Weise aufsprühen. Auf diese Weise erhält man Pellets, die im Kern das Öl und das Astaxanthin-Derivat enthalten.

## Patentansprüche

1. Formulierung eines Astaxanthin-Derivats, umfassend eine kontinuierliche Ölphase wenigstens eines für Nahrungsmittel geeigneten Öls und eine in der Ölphase emulgierte wässrige Phase, in der das Astaxanthin-Derivat in Form dispergierter, durch wenigstens ein Schutzkolloid stabilisierter Partikel vorliegt, wobei das Astaxanthin-Derivat zu wenigstens 70 Gew.-% aus wenigstens einer Verbindung der allgemeinen Formel I 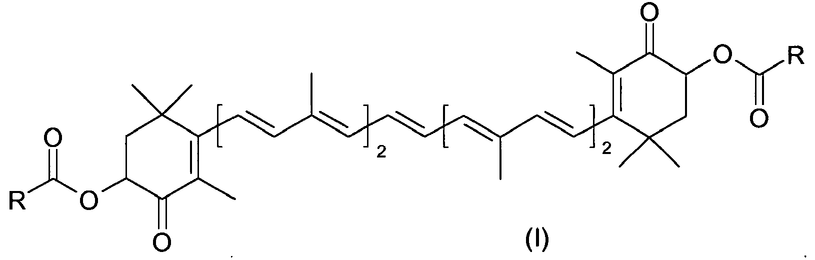 besteht, worin R für einen Rest der Formel 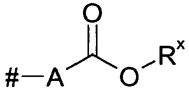 steht, worin # die Anknüpfung an die Carbonylgruppe bedeutet, A für -CH₂CH₂- oder für -CH=CH- steht und R^{x} für C₁-C₄ Alkyl steht.

2. Formulierung nach Anspruch 1, wobei in Formel I der Rest R^{x} Methyl oder Ethyl bedeutet und A für -CH₂CH₂- steht.

3. Formulierung nach Anspruch 1 oder 2, worin die durch das Schutzkolloid stabilisierten Partikel einen mittleren Teilchendurchmesser (Gewichtsmittel) im Bereich von 10 nm bis 2000 nm aufweisen.

4. Formulierung nach einem der vorhergehenden Ansprüche, worin das Schutzkolloid ausgewählt ist unter proteinischen Schutzkolloiden, Ligninsulfonsäuren, deren Salzen, Oligo- und Polysacchariden, Polyvinylalkohol, Polyvinylpyrrolidon und deren Gemischen.

5. Formulierung nach einem der vorhergehenden Ansprüche, enthaltend das Astaxanthin-Derivat in einer Menge von 0,1 bis 10 Gew.-%, bezogen auf das Gesamtgewicht der Zusammensetzung.

6. Formulierung nach einem der vorhergehenden Ansprüche, enthaltend einen Weichmacher in einer Menge von 1 bis 20 Gew.-%, bezogen auf das Gesamtgewicht der Zusammensetzung.

7. Verfahren zur Herstellung einer Formulierung nach einem der vorhergehenden Ansprüche, umfassend:
a) Bereitstellung einer wässrigen Dispersion des Astaxanthin-Derivats, das zu wenigstens 70 Gew.-% aus wenigstens einer Verbindung der allgemeinen Formel I besteht, wobei die wässrige Dispersion wenigstens ein Schutzkolloid enthält; und
b) Emulgieren der wässrigen Dispersion des Astaxanthin-Derivats in wenigstens einem für Nahrungsmittel geeigneten Öl.

8. Verfahren nach Anspruch 7, wobei die Bereitstellung der wässrigen Dispersion des Astaxanthin-Derivats die folgenden Schritte umfasst:
a1) Lösen des Astaxanthin-Derivats in einem organischen Lösungsmittel, das aus Kohlenstoff, Wasserstoff und Sauerstoff besteht,
a2) Vermischen der in Schritt a1) erhaltenen Lösung mit einer wässrigen Zusammensetzung, die wenigstens ein Schutzkolloid in gelöster Form enthält.

9. Verwendung einer Formulierung nach einem der Ansprüche 1 bis 6 zur Herstellung von Futtermittelzubereitungen, insbesondere festen Futtermittelzubereitungen, speziell Futtermittelzubereitungen in Form von Pellets, oder zur Herstellung von Lösungen des Astaxanthin-Derivats in einem für Nahrungsmittel geeigneten Öl.

10. Verfahren zur Herstellung von Futtermittelzubereitungen, insbesondere von festen Futtermittelzubereitungen, speziell von Pellets, umfassend:
α) Bereitstellung einer Zusammensetzung gemäß einem der Ansprüche 1 bis 6;
ß) Einarbeiten der Zusammensetzung in ein für Nahrungsmittel geeignetes, flüssiges Öl unter Erhalt einer verdünnten flüssigen, das Astaxanthin-Derivat enthaltenden Zusammensetzung in Form einer flüssigen Lösung des Astaxanthins in dem flüssigen Öl; und
γ) Vermischen der in Schritt ß) erhaltenen flüssigen Zusammensetzung mit den Bestandteilen der Futtermittelzubereitung oder Imprägnieren einer festen Futtermittelzubereitung mit der in Schritt ß) erhaltenen flüssigen Zusammensetzung.

## Claims

1. A formulation of an astaxanthin derivative comprising a continuous oil phase of at least one oil suitable for foods and an aqueous phase emulsified in the oil phase, in which aqueous phase the astaxanthin derivative is present in the form of dispersed particles stabilized by at least one protective colloid, wherein the astaxanthin derivative comprises at least 70% by weight of at least one compound of the general formula I 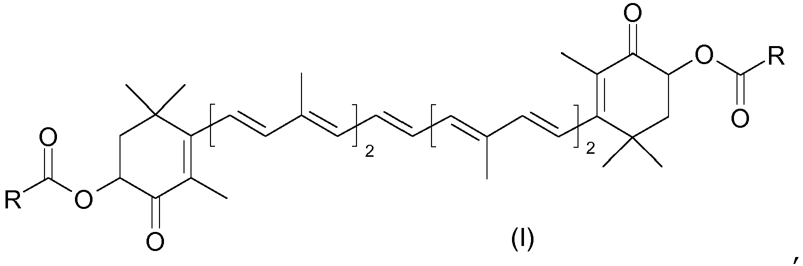 wherein R is a radical of the formula 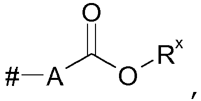 wherein # means the linkage to the carbonyl group, A is -CH₂CH₂- or -CH=CH- and R^{x} is C₁-C₄ alkyl.

2. The formulation according to claim 1, wherein, in formula I, the radical R^{x} is methyl or ethyl and A is -CH₂CH₂-.

3. The formulation according to claim 1 or 2, wherein the particles stabilized by the protective colloid have a median particle diameter (weight average) in the range from 10 nm to 2000 nm.

4. The formulation according to any one of the preceding claims, wherein the protective colloid is selected from proteinaceous protective colloids, lignosulfonic acids, salts thereof, oligosaccharides and polysaccharides, poly(vinyl alcohol), polyvinylpyrrolidone and mixtures thereof.

5. The formulation according to any one of the preceding claims, comprising the astaxanthin derivative in an amount of 0.1 to 10% by weight, based on the total weight of the composition.

6. The formulation according to any one of the preceding claims, comprising a plasticizer in an amount of 1 to 20% by weight, based on the total weight of the composition.

7. A process for producing a formulation according to any one of the preceding claims, which comprises:
a) providing an aqueous dispersion of the astaxanthin derivative which comprises at least 70% by weight of at least one compound of the general formula I, wherein the aqueous dispersion comprises at least one protective colloid; and
b) emulsifying the aqueous dispersion of the astaxanthin derivative in at least one oil suitable for foods.

8. The process according to claim 7, wherein providing the aqueous dispersion of the astaxanthin derivative comprises the following steps:
a1) dissolving the astaxanthin derivative in an organic solvent that comprises carbon, hydrogen and oxygen,
a2) mixing the solution obtained in step a1) with an aqueous composition that comprises at least one protective colloid in dissolved form.

9. The use of a formulation according to any one of claims 1 to 6 for producing feed preparations, in particular solid feed preparations, especially feed preparations in the form of pellets, or for producing solutions of the astaxanthin derivative in an oil suitable for foods.

10. A process for producing feed preparations, in particular solid feed preparations, especially pellets, which comprises:
α) providing a composition according to any one of claims 1 to 6;
ß) incorporating the composition into a liquid oil suitable for foods, obtaining a dilute liquid composition comprising the astaxanthin derivative in the form of a liquid solution of the astaxanthin in the liquid oil; and
γ) mixing the liquid composition obtained in step ß) with the components of the feed preparation or impregnating a solid feed preparation with the liquid composition obtained in step ß).

## Revendications

1. Formulation d'un dérivé d'astaxanthine, sous forme d'une composition comprenant une phase huileuse continue d'au moins un huile appropriée pour des aliments et d'une phase aqueuse émulsionnée dans la phase huileuse, dans laquelle le dérivé d'astaxanthine est présent sous forme de particules dispersées, stabilisées par au moins un colloïde protecteur, le dérivé d'astaxanthine consistant à raison d'au moins 70 % en poids en au moins un composé de formule générale I 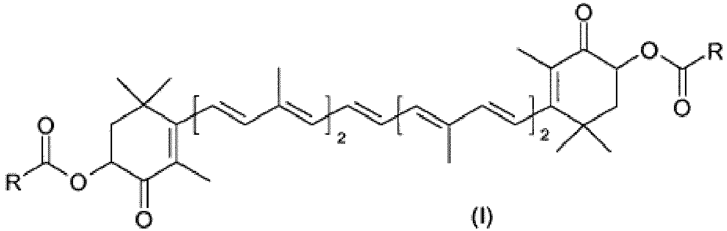 dans laquelle R représente un radical de formule 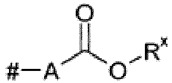 dans laquelle # représente l'attachement au groupe carbonyle, A représente -CH₂CH₂- ou -CH=CH- et R^{x} représente un groupe alkyle en C₁-C₄.

2. Composition selon la revendication 1, dans laquelle le radical R^{x} dans la formule I représente le groupe méthyle ou éthyle et A représente -CH₂CH₂-.

3. Composition selon la revendication 1 ou 2, dans laquelle les particules stabilisées par le colloïde protecteur ont un diamètre moyen (moyenne en poids) de particule dans la plage de 10 nm à 2 000 nm.

4. Composition selon l'une quelconque des revendications précédentes, dans laquelle le colloïde protecteur est choisi parmi des colloïdes protecteurs protéiques, des acides ligninesulfoniques, leurs sels, des oligo- et polysaccharides, le poly(alcool vinylique), la polyvinylpyrrolidone et des mélanges de ceux-ci.

5. Composition selon l'une quelconque des revendications précédentes, contenant le dérivé d'astaxanthine en une quantité de 0, 1 à 10 % en poids, par rapport au poids total de la composition.

6. Composition selon l'une quelconque des revendications précédentes, contenant un plastifiant en une quantité de 1 à 20 % en poids, par rapport au poids total de la composition.

7. Procédé pour la production d'une composition selon l'une quelconque des revendications précédentes, comprenant :
a) la préparation d'une dispersion aqueuse du dérivé d'astaxanthine qui consiste à raison d'au moins 70 % en poids en au moins un composé de formule générale I, la dispersion aqueuse contenant au moins un colloïde protecteur ; et
b) l'émulsification de la dispersion aqueuse du dérivé d'astaxanthine dans au moins une huile appropriée pour des aliments.

8. Procédé selon la revendication 7, dans lequel la préparation de la dispersion aqueuse du dérivé d'astaxanthine comprend les étapes suivantes :
a1) dissolution du dérivé d'astaxanthine dans un solvant organique qui est composé de carbone, hydrogène et oxygène,
a2) mélange de la solution obtenue dans l'étape a1) avec une composition aqueuse qui contient au moins un colloïde protecteur sous forme dissoute.

9. Utilisation d'une composition selon l'une quelconque des revendications 1 à 6, pour la fabrication de préparations d'aliments pour animaux, notamment de préparations solides d'aliments pour animaux, en particulier de préparations d'aliments pour animaux sous forme de boulettes ou pour la production de solutions du dérivé d'astaxanthine dans une huile appropriée pour des aliments.

10. Procédé pour la fabrication de préparations d'aliments pour animaux, notamment de préparations solides d'aliments pour animaux, en particulier de boulettes, comprenant :
α) la préparation d'une composition selon l'une quelconque des revendications 1 à 6 ;
β) l'incorporation de la composition dans une huile liquide appropriée pour aliments, avec obtention d'une composition contenant le dérivé d'astaxanthine, sous forme d'une solution liquide de l'astaxanthine dans l'huile liquide ; et
γ) le mélange de la composition liquide obtenue dans l'étape β) avec les composants de la préparation d'aliment pour animaux ou l'imprégnation d'une préparation solide d'aliment pour animaux avec la composition liquide obtenue dans l'étape β).
